# EUROPEAN PATENT APPLICATION

(11) **EP 4 473 943 A1**
(43) Date of publication of application: **11.12.2024**
(21) Application number: 23305885.8
(22) Date of filing: 05.06.2023
(51) Int. Cl.: A61F 2/24

(54) **MITRAL VALVE PROSTHETIS**

(71) Applicant: Educat, 92110 Clichy (FR)
(72) Inventor: MOUSSAFEUR, Amina, 92110 Clichy (FR)
(74) Representative: Icosa

(57) **Abstract**

Mitral valve prosthesis frame (10) configured to be positioned inside an opening (105) of a native mitral valve (103) of a patient's heart (100), said frame comprising:
- a ventricle part (12) extending along a first axis (X₁), the ventricle part being configured to be positioned at least partially inside the left ventricle (LV) of the patient's heart,
- an atrial part (14) extending along a second axis (X₂), the atrial part being configured to be positioned at least partially inside the left atrium (LA) of the patient's heart.

The ventricle part comprises at least one securing element (16) configured to secure the native leaflets (107) to the ventricle part. The atrial part comprises a flexible portion (15) connected to the ventricle part, said flexible portion allowing an angle (α) between the first and second axis to be variable.

## Description

### FIELD OF INVENTION

The invention relates to mitral valve replacement prostheses. More particularly of the type of percutaneously implantable prostheses.

### BACKGROUND OF INVENTION

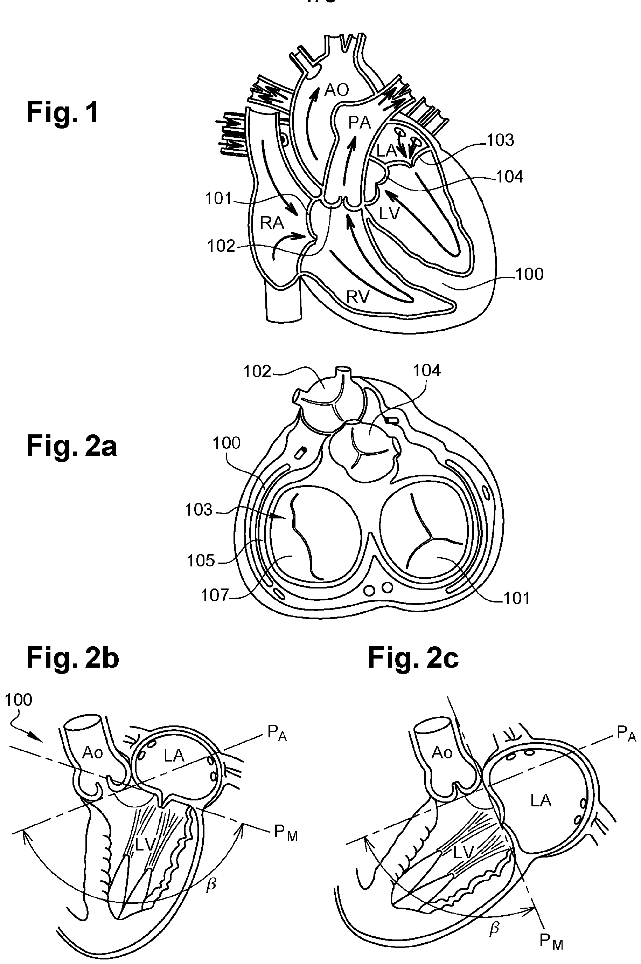

As can be seen on figure 1, considering a classic human heart 100, oxygen-poor blood enters the heart 100 through the right atrium RA, crosses the tricuspid valve 101, enters the right ventricle RV, crosses the pulmonary valve 102 into the pulmonary artery PA and moves on to the lungs. Oxygen-rich blood comes back from the lungs into the heart 100 through the left atrium LA, crosses the mitral valve 103, enters the left ventricle LV, eventually crosses the aortic valve 104 and leaves the heart through the aorta AO.

As can be seen more precisely on figure 2a, anatomically, the mitral and aortic valves 103, 104 are in fibrous continuity. As can be seen on figure 2b, the mitral valve 103 presents an opening 105 (or mitral annulus) through which the blood can flow and which can be closed/opened by two native leaflets 107. The mitral valve 103 thus presents a mitral annular plane P_{M} containing the mitral annulus (opening) 105. Similarly, the aortic valve 104 presents an opening (aortic annulus) also called left ventricular outflow tract (LVOT) through which the blood can flow and which can be closed/opened by three native leaflets 107. The aortic valve 104 thus presents an aortic annular plane Ap containing said aortic annulus.

As can be seen on figure 2b, the mitral annular plane P_{M} and the aortic plane P_{A} form an angle β known as the aortomitral angle. Said angle β can vary from one individual to another, depending on the specific anatomy of the patient (see figure 2c).

Mitral valve regurgitation is the second valvular disease that affects 1 to 2% of the adult population. In economically developed countries, the three most frequent causes of mitral valve regurgitation are mitral valve prolapsus, functional mitral regurgitation and mitral annulus mitral calcification (MAC). Mitral valve repair is feasible for prolapsus and functional etiology when the leaflets are not calcified and/or the coaptation gap is not too large. Mitral valve replacement is required when the prolapsus is complex, in case of calcification or large coaptation gap.

It is well known in the state of the art, that one of the known potential complications of mitral percutaneous valve replacement is obstruction of the left ventricular outflow tract LVOT by the newly implanted valve prosthesis.

LVOT obstruction following mitral valve replacement can occur by several mechanisms.

One well-known of the mechanism is the that the placement of a prosthetic valve inside the mitral annulus results in projection of said prosthesis into the left ventricle LV in close proximity to the aortic annulus. The aortomitral angle α between the annular plane of the aortic and mitral valves P_{A}, P_{M}, described here-above has a direct impact on the degree of protrusion of the mitral prosthesis into the LVOT. The closer the aortomitral angle β is to 180°, the less potential obstruction of the LVOT there is. The risk of LVOT obstruction is highest when the aortomitral angle β is 90° (figure 2c), when the LVOT and mitral annulus are perpendicular to one another.

The second factor of obstruction is the length of the anterior leaflet that covers the prosthetic valve frame and prevents the blood flowing out to the LVOT.

For a number of patients (40-60%) with different cardiac anatomies or pathologies, the aortomitral angle β is too small (<125°) and the length of the anterior leaflet is too large (>25mm), which results in aortic valve occlusion when a prosthesis is implanted inside the mitral annulus. Therefore, these patients are usually not offered a mitral valve prosthesis.

Most of these patients denied from surgery and percutaneous valve implantation will experience repetitive heart failure and likely a premature death.

The present invention therefore offers a solution for those patients in addressing this high risk LVOT obstruction.

### SUMMARY

This invention thus relates to a mitral valve prosthesis frame configured to be positioned inside an opening of a native mitral valve of a patient's heart, said frame comprising:
- a ventricle part extending along a first axis, the ventricle part being configured to be positioned at least partially inside the left ventricle of the patient's heart,
- an atrial part extending along a second axis, the atrial part being configured to be positioned at least partially inside the left atrium of the patient's heart,

**wherein** the ventricle part comprises at least one securing element configured to secure the native leaflets to the ventricle part,
   **wherein** the atrial part comprises a flexible portion connected to the ventricle part, said flexible portion allowing an angle between the first and second axis to be variable.

Thus, this solution achieves the above objective. In particular, it allows limiting the risk of LVOT by tilting the implantation angulation with regards to a natural axis of the native mitral valve. More particularly, the flexible portion allows the tilting of the implantation angulation towards the posterior wall of the patient's heart.

The device according to the invention may include one or more of the following characteristics, taken in isolation from one another or in combination with one another:
- the at least one securing element of the ventricle part may be configured to secure and confine the native leaflets at a distance ranging from 8 to 12mm, preferably of 10mm below the opening of a native mitral valve,
- the first and second axis and may not be parallel and constantly form a non-zero angle, so that the ventricle part is permanently tilted with respect to the atrial part,
- the angle between the first and second axis may ranges from 0° to 45°, preferably from 10° to 30°,
- the ventricle part may present an external and an internal face, the native leaflets being secured on the external face of the ventricle part when the frame is implanted,
- the native leaflets being secured and confined on the external face of the ventricle part when the frame is implanted,
- the native leaflets are secured on the external face of the ventricle part around 10mm below the mitral annulus when the frame is implanted,
- the frame may comprise a plurality of securing elements distributed around the ventricle part, each securing element comprising an elongated member extending from an extremity of the ventricle part and being configured to bear against the external face of the ventricle part in order to catch and confine the native leaflets between at least one elongated member and the external face of the ventricle part, when the frame is implanted inside the patient's heart,
- the introducer system used to open and close the securing elements distributed around the ventricle is designed to confine the leaflets at 10mm below the annulus level,
- each elongated member may present a free end and at least one immobilization element near said free end, configured to be pushed inside the native leaflets in order to improve the securing of the native leaflets to the ventricle part,
- the ventricle part may comprise, on its external face, at least one hooking or piercing element to improve securing of the native leaflets to the ventricle part,
- the flexible portion may comprise zig zag shaped wires,
- the ventricle part may present a conical shape with a greater upstream diameter ranging from 25 to 55mm and a smaller downstream diameter ranging from 20 to 45mm,
- the ventricle part may present a length ranging from 20 to 30mm,
- the ventricle part is an open stent partly covered,
- the ventricle part may be configured, when implanted, to cooperate radially with the opening of the native mitral valve,
- the ventricle part and the flexible portion may cooperate to ensure, when implanted, a maximal radial cooperation of the ventricle part with the opening, in order to achieve a sealed radial contact between the opening and the ventricle part,
- the atrial part may present a general conical shape with an upstream diameter ranging from 45 to 65mm, and a downstream diameter ranging from 25 to 55 mm,
- the atrial part may present a length ranging from 5 to 15 mm,
- the second axis may present, when the frame is implanted, an angle of around 90° with a mitral annular plane of the native mitral valve opening,
- the frame may be made of a compressible material, preferably a memory shape alloy material, more preferably of nitinol,
- the frame may be made of a meshed material.
- the frame may further comprise at least one marker detectable by X-ray or ultrasound imaging to ease orientation of the frame during implantation,
- the atrial part may further comprise coupling members to secure the frame to a catheter during an implantation method.

Another object of the invention is about a mitral valve prosthesis comprising a mitral valve prosthesis frame as described here-above and prosthetic leaflets secured inside the ventricle part of the frame and configured to replace the function of native mitral valve leaflets.

A further object of the present invention is about a method for implanting a mitral valve prosthesis as described here-above, in a patient's heart the method comprising the steps of:
- providing a catheter assembly comprising a catheter and the mitral valve prosthesis in a retracted configuration inside the said catheter,
- percutaneously inserting the catheter assembly inside the patient's left ventricle through the mitral valve opening of the patient's heart,
- advancing the mitral valve prosthesis inside the catheter in order to deploy the at least one securing element,
- catching and confining at least one leaflet of the native mitral valve with the deployed securing element,
- further advancing the mitral valve prosthesis inside the catheter in order to release and position the whole mitral valve prosthesis inside the mitral valve opening of the patient's heart.

### BRIEF DESCRIPTION OF THE DRAWINGS

The invention will be better understood, and other aims, details, characteristics and advantages thereof will emerge more clearly on reading the detailed explanatory description which follows, of embodiments of the invention given by way of illustration. purely illustrative and non-limiting examples, with reference to the accompanying drawings:
- **figure 1** is a schematical open view of a classic human heart,
- **figure 2a** is a schematical axial section view of a human heart showing the valves,
- **figure 2b** is a schematical frontal section view of a human heart showing the mitral and aortic valves of a first patient,
- **figure 2c** is a schematical frontal section view of a human heart showing the mitral and aortic valves of a second patient,
- **figure 3** is a perspective view of half a frame according to the present invention,
- **figure 4** is a schematic view of a mitral prosthesis kit according to the present invention implanted inside a patient's heart,
- **figure 5** is a view from above of a frame according to the present invention before its 3D expansion,
- **figure 6** is a schematic longitudinal view of the frame according to the present invention,
- **figure 7** is a step-by-step schematical view of the implantation method according to the present invention,
- **figure 8** is a similar view to figure 7 of another embodiment of the present invention.

### DETAILED DESCRIPTION

### Functional description

ln order to enable a percutaneous implantation (see further below for more detailed description of the implantation method), the mitral valve prosthesis frame 10 according to the present invention, is made of a compressible material, preferably a memory shape alloy material, more preferably of nitinol. This compressible material makes it possible for the frame 10 to expand once it has been implanted inside the patient's heart 100.

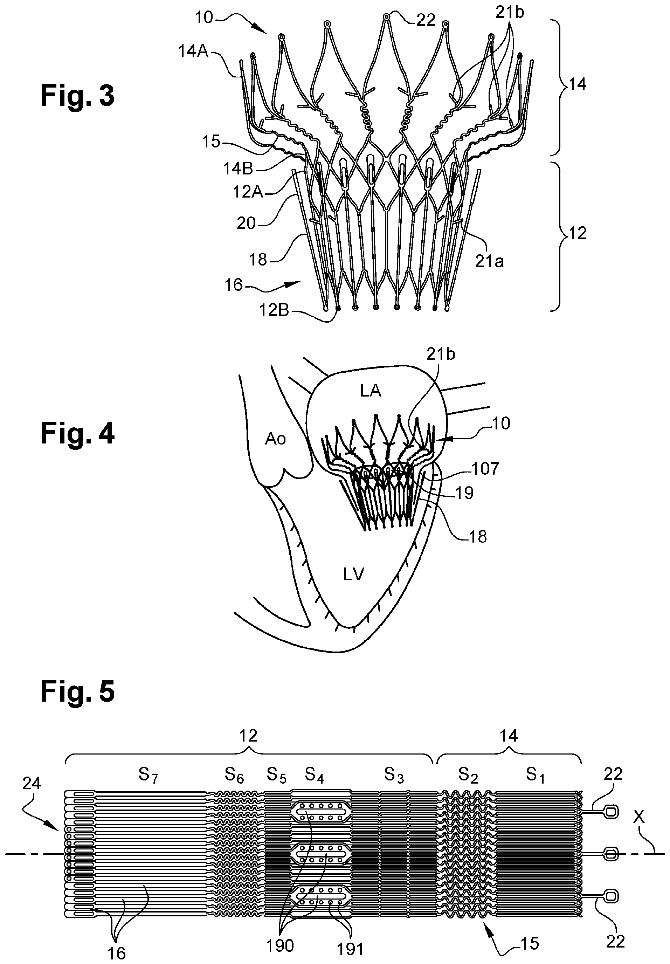

On the embodiments represented on figures 3 and 4, the frame 10 is made of a meshed material, which can easily be compressed for the implantation purposes and can easily and safely expand within the patient's heart 100, once implanted.

As can be seen on figures 3 to 6, this invention relates to a mitral valve prosthesis frame 10 configured to be positioned inside an opening (or mitral annulus) 105 of a native mitral valve of a patient's heart 100.

The frame 10 according to the present invention comprises:
- a ventricle (or downstream) part 12 extending along a first axis X₁,
- an atrial (or upstream) part 14 extending along a second axis X₂ forming an angle α with the first axis X₁, the atrial part 14 further comprising a flexible portion 15 connected to the ventricle part 12.

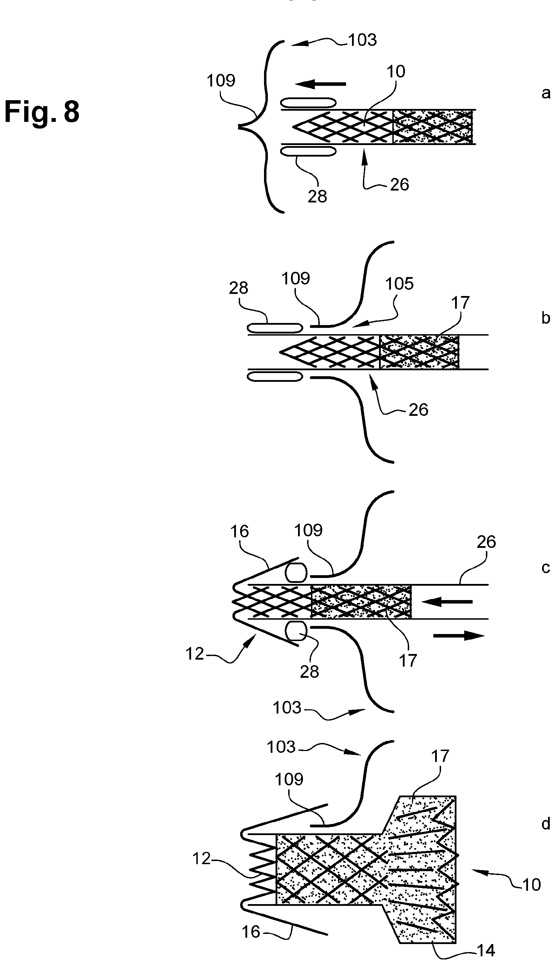

In some embodiments, the ventricle part 12 and the atrial part 14 are coated with a membrane of porcine pericardial tissue 17, as can be seen on the embodiment depicted on figure 8. This enables the frame 10 to merge with the biological walls of the native mitral valve 103 of the patient's heart 100 and avoid rejection reactions. In particular, the ventricle part 12 may typically be is an open stent partly covered (or coated) with said membrane starting from around 10mm below the atrial part.

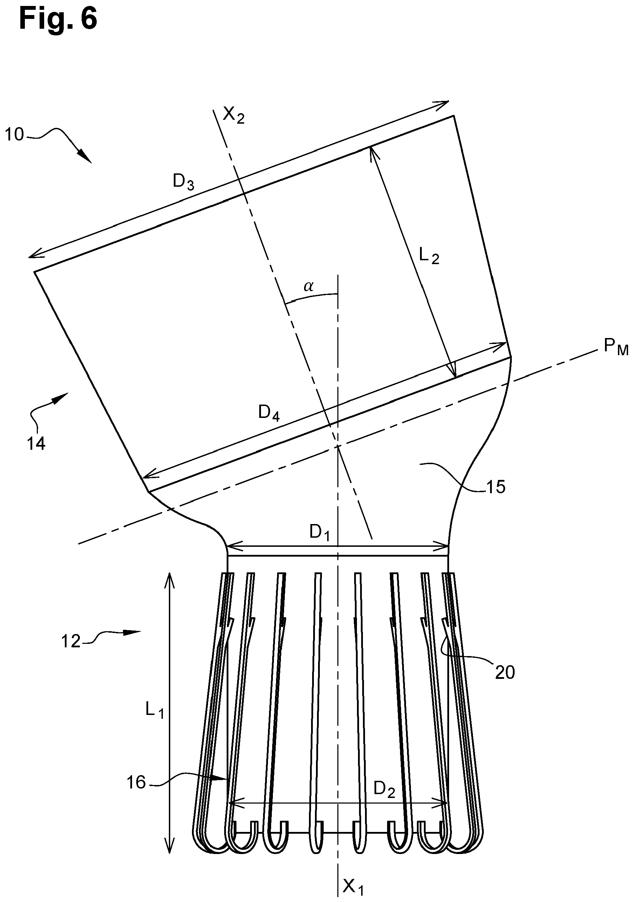

When expanded, as can be seen on figures 4 and 6, the ventricle part 12 of the frame 10 presents a conical shape with a greater atrial (upstream) diameter D₁ ranging from 25 to 55mm and a smaller ventricular (downstream) diameter D₂ ranging from 20 to 45mm. The conical shape prevents valve squishing in patients with small left ventricle. The ventricle part 12 presents a length Li ranging from 20 to 30mm, in order to prevent interaction with papillary muscles.

As can be seen on figure 4, the ventricle part 12 is configured to be positioned at least partially inside the left ventricle LV of the patient's heart 100. As can be seen on figure 4, the ventricle part 12 is configured, when implanted inside the patient's heart 100, to cooperate radially with the opening 105 of the native mitral valve 103. The ventricle part 12 thus presents an upstream extremity 12A connected to the atrial part 14 of the frame 10 and a free downstream extremity 12B. The free downstream extremity 12B of the ventricle part 12 of the frame is configured to be located inside the left ventricle of the patient's heart 100.

As can be seen on figure 3, the ventricle part 12 of the frame 10 comprises at least one securing element 16 configured to secure and to preferably confine the native leaflets 107 to the ventricle part 12. Preferably, the ventricle part 12 of the frame 10 comprises four to nineteen securing elements 16. More precisely, as the ventricle part 12 presents two faces (an external face and an internal face), the native leaflets 107 are to be secured on the external face of the ventricle part 12 when the frame 10 is implanted.

Preferably, the frame 10 is implanted in such a way that the native leaflets 107 are secured around 10mm below the mitral annulus (opening) 105. Preferably, the at least one securing element 16 of the ventricle part 12 is configured to secure and confine the native leaflets 107 at a distance ranging from 8 to 12mm, preferably of 10mm below the opening 105 (or annulus) of the native mitral valve 103. This securing of the native leaflets 107 around 10mm below the mitral opening 105 allows limiting the risk of LVOT obstruction by limiting the native leaflets 107 covering more than 10mm of the mitral opening 105. Confining the native leaflet 107 at around 10mm below the annulus level is one important feature for preventing LVOT obstruction. This way, once the frame 10 is implanted inside the opening 105 of the mitral valve 103, the native leaflets 107 are safely secured to the ventricle part 12 and cannot get stuck or trapped in an inappropriate place, putting the heart 100 at risk to be harmed or obstructing the right functioning of the implanted prosthesis.

In order to achieve said securing of the native leaflets 107, the frame 10 according to the present invention comprises a plurality of securing elements 16 distributed (preferably regularly distributed) around the ventricle part 12. A homogeneous or regular distribution of those securing elements 16 around the ventricle part 12 enables to reduce the pressure exerted during the systolic blood ejection. Each securing element 16 of the ventricle part 12 comprises an elongated member 18 (for example a rod) extending from one of the extremities 12A, 12B of the ventricle part 12. Each of those elongated members 18 presents a length ranging from 20 to 30 mm and a width ranging from 1 to 2mm.

In order to better accommodate the natural shape of the native leaflets 107, each elongated member 18 extends from the free downstream extremity 12B of the ventricle part 12. Preferably, each elongated member 18 extends from the free downstream end 12B of the ventricle part 12 towards the upstream extremity 12A of said ventricle part 12. Preferably, the distance d between the free end of each elongated member 18 and the upstream extremity 12A of the ventricle part 12 ranges from 8 to 12mm, and is preferably of 10mm (see figure 8d). This enables to secure the native leaflets 107 around 10mm below the mitral annulus (opening) 105, once the frame 10 is implanted.

Each of those securing elements 16 is further configured to bear against the external face of the ventricle part 12. However, those securing elements 16 are also elastic enough to be slightly pulled away from the external face of the ventricle part 12 such that, when the frame 10 is implanted, the native leaflets 107 can be caught between the at least one elongated member 18 and the external face of the ventricle part 12. The distance d between the free end of each elongated member 18 and the upstream extremity 12A of the ventricle part 12 enables to safely and comfortably catch and confine the native leaflets 107 without risking to hurt them.

In order to improve the securing of the native leaflets 107 when the frame 10 is implanted, each elongated member 18 presents a free end and at least one immobilization element 20 near said free end. This at least one immobilization element 20 can for example, present the shape of a hook or a point. Each immobilization element 20 is thus configured to be pushed inside the native leaflets 107 in order to improve the securing of the native leaflets 107 to the ventricle part 12 of the frame 10.

In some embodiments, the ventricle part 12 comprises on its external face at least one secondary leaflet hooking or piercing element 21a of about 2mm length (see figure 3). The ventricle 12 preferably comprises four to nineteen elements regularly disposed on its circumference. The purpose of those secondary leaflets hooking or piercing elements 21a is to even further improve the securing of the native leaflets 107 to the ventricle part 12. This way, the native leaflets 107 are secured both to the external face of the ventricle part 12 and to the elongated member 18, ensuring a very strong attachment.

In order to replace the function of the native mitral valve leaflets 107, once the frame 10 is implanted and secured to them, the frame 10 is configured to receive and maintain a series of prosthetic leaflets 19, preferably three prosthetic leaflets 19. Those prosthetic leaflets 19 are thus secured inside the ventricle part 12 of the frame 10 and configured to replace the function of the native leaflets 107 of the native mitral valve 103. The prosthetic leaflets 19 are for example made of porcine pericardial tissue. Each prosthetic leaflet 19 presents a rectangular shape with dimensions ranging from 15 to 25mm, preferably 26 x 20mm.

The immobilization elements 20 are preferably located, when the frame 10 is implanted, at a distance ranging from 5 to 10 mm from the mitral plane P_{M}. The hooking or piercing elements of the external face of the ventricle part 12 are, when present on the embodiment, also preferably located, when the frame 10 is implanted, at a distance ranging from 5 to 10 mm from the mitral plane P_{M}.

As can be seen on figure 4, the atrial part 14 is configured to be positioned at least partially inside the left atrium LA of the patient's heart 100.

When expanded, the atrial part 14 presents a general conical shape with an upstream (atrial) diameter D₃ ranging from 45 to 65mm, and a downstream (ventricle) diameter D₁ ranging from 25 to 55 mm. The atrial part 14 thus forms a circular flange or collar. The atrial part 14 presents a length L₂ ranging from 5 to 15 mm. The atrial part 14 presents a larger upstream diameter D₃ than the ventricular part 12 in order to enable it to securely cling to the inside of the left atrium LA of the patient's heart 100 and avoid its descent into the left ventricle LV, through the opening 105 of the mitral valve 103.

In some embodiments, the atrial part 14 comprises on its perimeter at least one atrium wall hooking or piercing element 21b (see figure 3) of about 1 to 2mm length. The atrial part 14 preferably comprises 4 to 20 elements. The purpose of those atrium wall hooking or piercing elements 21b is to improve the securing of the frame 10 inside the left atrium LA of the patient's heart 100 and thus further improve its global stability over time. Those hooking or piercing elements 21b form an anchoring system to prevent the frame 10 from tilting or slandering.

In order to enable an easy implantation, in some embodiments, the atrial part 14 further comprises coupling members 22 to secure the entire frame 10 to a catheter during an implantation procedure involving an implantation method (see further below).

In order to correctly position the frame 10 inside the patient's heart 100 and maximize the blood circulation through the prosthetic mitral valve, it is necessary that the second axis X₂ presents, when the frame 10 is implanted, an angle of around 90° with the mitral annular plane P_{M} of the native mitral valve 103.

As mentioned above, the atrial part 14 comprises a flexible portion 15 connected to the ventricle part 12. More precisely, the atrial part 14 comprises a free upstream extremity 14A and a downstream extremity 14B comprising the flexible portion 15 connected to the upstream extremity 12A of the ventricle part 12. This flexible portion 15 can for example be made of an ensemble of very elastic strings distributed on the circumference of the atrial part 15. In the represented embodiment on figure 3, said flexible portion 15comprises as series of zig zag shaped wires. Those zigzag shaped wires can easily vary in length and be bent, if necessary. In another embodiment, not shown, the flexible portion 15 could present a thinning of the wires of the meshed material the frame is made of, or a piston made of another material, for example.

More specifically, the ventricle part 12 and the flexible portion 15 are configured to cooperate together, as can be understand from figure 6, to ensure, when implanted, a maximal radial cooperation of the ventricle part 12 with the opening 105 of the native mitral valve 103, in order to achieve a sealed radial contact between the opening 105 and the ventricle part 12. Such a sealed contact is very important to ensure the long-term fitting and maintained position of the prosthesis inside the opening 105 of the native mitral valve 103.

Another effect of the deformable portion 15 is to take back the efforts exerted, by the flooding blood, on the ventricle part 12 (especially on the prosthetic leaflets 19 carried by said ventricle part 12). This contributes to avoid any long-term displacement of the frame 10 in the left ventricle LV of the patient's heart 100.

The flexible portion 15 of the atrial part 14 further allows the angle α between the first axis X₁ and second axis X₂ axis to be variable. This enables an adaptation of the frame 10 to the specific anatomy of each patient's heart 100, particularly regarding the aortomitral angle β of said patient's heart 100. In order to be adapted to the aortomitral angle β of each patient's heart 100, the angle a between the first and second axis X₁, X₂, ranges from 0° to 45°, preferably from 10° to 30°.

In order to further improve said adaptability and to avoid any unwanted damage to the frame 10 by a too huge solicitation of the deformable portion 15 during implantation, in some embodiments, the first and second axis X₁ and X₂ are not parallel, even before implantation, and constantly form a non-zero angle α, so that the ventricle part 12 is permanently tilted with respect to the atrial part 14.

As the frame 10 is not symmetrical by construction, its introduction inside the patient's heart 100 necessitates to know its orientation at all times during the implantation process.

In order to achieve this, the mitral valve prosthesis frame 10 according to the present invention, further comprises at least one marker 24 detectable by X-ray or ultrasound imaging to ease orientation of the frame during implantation, as can be seen on figure 6. This marker 24 or radio-tag could thus be used to identify, in real time, the "front" and "back" of the frame 10. In some embodiments, the markers are placed at the ends of the elongated members 18. This enables to couple geometric and structural marking and thus allows visual identification during the loading of the frame 10 into a catheter, on ultrasound and X-ray.

### Structural description

As can be seen on figure 5, before being wrapped and expanded, the frame 10 according to the present invention, presents as a general square shaped meshed piece of material extending along a global longitudinal axis X. It comprises a series of wires, all globally aligned along the global longitudinal axis X.

Along this longitudinal axis X, the frame 10 presents a striped structure, each stripe S₁, S₂, S₃, S₄, S₅, S₆, S₇ presenting a specific meshed structure. Each stripe S₁, S₂, S₃, S₄, S₅, S₆, S₇ is configured to fulfill a specific technical function.

At its first extremity, corresponding to the upstream extremity 14A of the atrial part 14, the frame 10 presents a series of loops. Those loops are the coupling members 22 configured to enable the coupling of the frame 10 to a catheter during the implantation process. The upstream extremity 14A of the ventricle part 14 is made of a first stripe S₁ of aligned longitudinal wires. Those longitudinal wires transform into waved, or zig-zag shaped wires for the second stripe S₂. The second stripe S₂ corresponds to the deformable portion 15 and presents, as already mentioned, for example, zig-zag shaped wires enabling the tilting of the ventricle part 12 with regards to the atrial part 14. The waved or zig-zag shaped wires further enable to give the atrial part 14 its flared conical shape. The third stripe S₃ corresponds to the upstream extremity 12A of the ventricle part 12. The third stripe S₃ comprises, similarly to the first stripe S₁, a series of aligned longitudinal wires, reinforced by transversal wires. The fourth stripe S₄ comprises large pieces of material each one presenting an aperture 190 surrounded by small stitching holes 191, in order to be able to secure the prosthetic leaflets 19 to the frame 10, preferably by means of stitching them to the frame 10. The fifth stripe S₅ presents a structure similar to the first and third stripes S₁, S₃. The sixth stripe S₆ presents thick wavy wires in order to allow bending the arms at 90° without stent fractures. The sevenths stripe S₇ corresponds to the free downstream extremity 12B of the ventricle part 12, which presents the same structure all along its length and carries the securing elements 16. The sevenths stripe S₇ present a series of wider wires, almost thin rods, in order to offer a strong resistance when securing and maintaining the native leaflets 107 once implanted.

### Implantation method

The here-above-described frame 10 is to be used to implement a method for implanting, inside a patient's heart 100, a mitral valve prosthesis comprising a frame 10 and a series of prosthetic leaflets 19. The method comprises following steps:
- providing a catheter assembly comprising a catheter 26 and the mitral valve prosthesis 10, 19 in a retracted configuration inside the said catheter 26,
- attaching the mitral valve prosthesis 10, 19 to the catheter 26 by means of the coupling members 22,
- percutaneously inserting the catheter assembly inside the patient's left ventricle LV through the mitral valve opening 105 of the patient's heart 100,
- advancing the mitral valve prosthesis 10, 19 inside the catheter 26 in order to deploy the at least one securing element 16,
- catching and confining at least one native leaflet 107 of the native mitral valve 103 with the deployed securing element 16,
- further advancing the mitral valve prosthesis 10, 19 inside the catheter 26 in order to release and position the whole mitral valve prosthesis 10, 19 inside the mitral valve opening 105 of the patient's heart 100.

More precisely, the first step of the implantation method implies the placement of a guidewire in the left ventricle LV via a well-known in the state of art, trans-septal approach using, for example, an *Agilis*^{®} deflectable probe (this step is not shown).

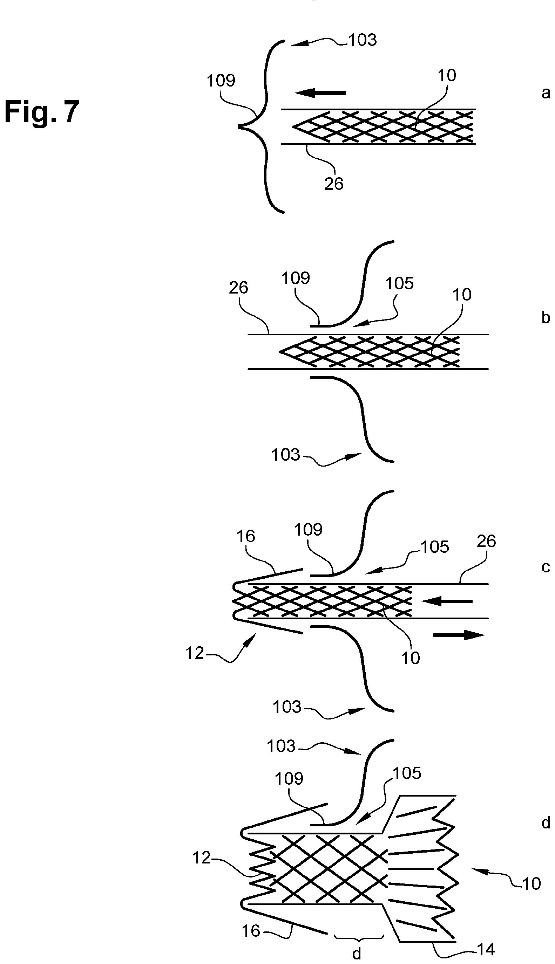

After the guidewire has been introduced, the method displays the introduction of an introducer and catheter 26 with the mitral valve prosthesis 10, 19 (the mitral valve prosthesis comprising the mitral valve prosthesis frame 10 secured to the prosthetic leaflets 19) folded inside. This ensemble formed by the catheter 26 enclosing the mitral valve prosthesis 10, 19 is then led to approach of the native mitral valve 103 (see step a of figure 7).

In some embodiments, as depicted on figure 8, the catheter 26 comprises, at its distal extremity, at least one balloon 28 which can be inflated on demand by the surgeon. Each balloon 28 is sized to confine the native mitral valve 103 to the area of the frame 10 covered with the membrane of porcine pericardial tissue 17.

The ensemble formed by catheter 26 enclosing the mitral valve prosthesis 10, 19 is then made to enter the opening 105 of the native mitral valve 103 in order to ensure the crossing of the native mitral 103 valve by the catheter 26 and enabling the hooking of the frame 10 inside the left ventricle LV of the patient's heart 100 (see step b of figure 7).

The catheter 26 is the pulled back, so that the ventricle part 12 of the mitral valve prosthesis 10, 19 sticks out and starts to expand, particularly the at least one securing element 16 (see step c of figure 7). Preferentially, the at least one securing element 16 is moved away from the catheter 26 and spread laterally (separated from the external face of the ventricle part 12 of the frame 10) by means, for example, of the balloon 28 (see figure 8c) located at the distal end of the catheter 26 if the catheter 26 comprises such a balloon 28. By spreading the at least one securing element 16, it enables the frame 10 to catch and confining the native leaflets 109 when the at least one securing element 16 fells back into place after removing the balloon.

Finally, the catheter 26 is fully removed from the patient's heart 100, the atrial part 14 of the frame 10 is freed and can also expand, leading the mitral valve prosthesis 10, 19 to be fully deployed on both sides of the opening 105 of the native mitral valve 103, in the left atrium LA and the left ventricle LV of the patient's heart 100 (see step of figure 7).

## Claims

1. Mitral valve prosthesis frame (10) configured to be positioned inside an opening (105) of a native mitral valve (103) of a patient's heart (100), said frame (10) comprising:
- a ventricle part (12) extending along a first axis (X₁), the ventricle part (12) being configured to be positioned at least partially inside the left ventricle (LV) of the patient's heart (100),
- an atrial part (14) extending along a second axis (X₂), the atrial part (14) being configured to be positioned at least partially inside the left atrium (LA) of the patient's heart (100),
**wherein** the ventricle part (12) comprises at least one securing element (16) configured to secure the native leaflets (107) to the ventricle part (12),
**wherein** the atrial part (14) comprises a flexible portion (15) connected to the ventricle part (12), said flexible portion (15) allowing an angle (α) between the first (X₁) and second (X₂) axis to be variable.

2. Mitral valve prosthesis frame (10) according to the preceding claims, wherein the at least one securing element of the ventricle part (12) is configured to secure and confine the native leaflets (107) at a distance ranging from 8 to 12mm, preferably of 10mm below the opening (105) of a native mitral valve (103).

3. Mitral valve prosthesis frame (10) according to anyone of the preceding claims, wherein the first and second axis (X₁) and (X₂) are not parallel and constantly form a non-zero angle (α), so that the ventricle part (12) is permanently tilted with respect to the atrial part (14).

4. Mitral valve prosthesis frame (10) according to the preceding claim, wherein the angle (α) between the first and second axis (X₁, X₂) ranges from 0° to 45°, preferably from 10° to 30°.

5. Mitral valve prosthesis frame (10) according to any one of the preceding claims, wherein the ventricle part (12) presents an external and an internal face, the native leaflets (107) being secured on the external face of the ventricle part (12) when the frame (10) is implanted.

6. Mitral valve prosthesis frame (10) according to the preceding claim, comprising a plurality of securing elements (16) distributed around the ventricle part (12), each securing element (16) comprising an elongated member (18) extending from an extremity (12A, 12B) of the ventricle part (12) and being configured to bear against the external face of the ventricle part (12) in order to catch and confine the native leaflets (107) between at least one elongated member (18) and the external face of the ventricle part (12), when the frame (10) is implanted inside the patient's heart (100).

7. Mitral valve prosthesis frame (10) according the preceding claim, wherein each elongated member (18) presents a free end and at least one immobilization element (20) near said free end, configured to be pushed inside the native leaflets (107) in order to improve the securing of the native leaflets (107) to the ventricle part (12).

8. Mitral valve prosthesis frame (10) according to any one of the preceding claims, wherein the ventricle part (12) comprises, on its external face, at least one secondary leaflet hooking or piercing element (21a).

9. Mitral valve prosthesis frame (10) according to any one of the preceding claims, wherein the ventricle part (12) presents a conical shape with a greater upstream diameter (Di) ranging from 25 to 55mm and a smaller downstream diameter (D₂) ranging from 20 to 45mm.

10. Mitral valve prosthesis frame (10) according to any one of the preceding claims, wherein the ventricle part (12) is configured, when implanted, to cooperate radially with the opening (105) of the native mitral valve (103).

11. Mitral valve prosthesis frame (10) according to the preceding claim, wherein the ventricle part (12) and the flexible portion (15) cooperate to ensure, when implanted, a maximal radial cooperation of the ventricle part (12) with the opening (105), in order to achieve a sealed radial contact between the opening (105) and the ventricle part (12).

12. Mitral valve prosthesis frame (10) according to the preceding claim, wherein the atrial part (14) presents a general conical shape with an upstream diameter (D₃) ranging from 45 to 65mm, and a downstream diameter (Di) ranging from 25 to 55 mm.

13. Mitral valve prosthesis frame (10) according to the any one of the preceding claims, wherein the atrial part (14) comprises, on a perimeter, at least one atrium wall hooking or piercing element (21b).

14. Mitral valve prosthesis frame (10) according to any of the preceding claims, further comprising at least one marker detectable by X-ray or ultrasound imaging to ease orientation of the frame (10) during implantation.

15. Mitral valve prosthesis frame (10) according to any of the preceding claims, wherein the atrial part (14) further comprises coupling members to secure the frame (10) to a catheter during implantation.

16. Mitral valve prosthesis (10, 19) comprising a mitral valve prosthesis frame (10) according to any one of the preceding claims and prosthetic leaflets (19) secured inside the ventricle part (12) of the frame (10) and configured to replace the function of native mitral valve leaflets (107).

17. Method for implanting a mitral valve prosthesis (10, 19) according to claim 14 in a patient's heart (100), the method comprising the steps of:
- providing a catheter assembly comprising a catheter and the mitral valve prosthesis (10, 19) in a retracted configuration inside the said catheter,
- percutaneously inserting the catheter assembly inside the patient's left ventricle (LV) through the mitral valve opening (105) of the patient's heart (100)
- advancing the mitral valve prosthesis (10, 19) inside the catheter in order to deploy the at least one securing element (16),
- catching and confining at least one leaflet of the native mitral valve with the deployed securing element (16),
- further advancing the mitral valve prosthesis (10, 19) inside the catheter in order to release and position the whole mitral valve prosthesis (10, 19) inside the mitral valve opening (105) of the patient's heart (100).
